# EUROPÄISCHE PATENTSCHRIFT

(11) **EP 2 392 253 B1**
(45) Veröffentlichungstag und Bekanntmachung des Hinweises auf die Patenterteilung: **17.06.2020**
(21) Anmeldenummer: 11004763.6
(22) Anmeldetag: 18.04.2008
(51) Int. Cl.: G06F 8/65, A61B 5/00, A61M 16/00

(54) **Vorrichtung und Verfahren zur Aktualisierung von Beatmungsgeräten**
Method and device for updating respirators
Dispositif et procédé destinés à l'actualisation d'appareils respiratoires

(30) Priorität: 18.04.2007 DE 102007018587
(43) Veröffentlichungstag der Anmeldung: 07.12.2011
(62) Teilanmeldung aus: 08400027.2
(73) Patentinhaber: Löwenstein Medical Technology S.A., 2557 Luxembourg (LU)
(72) Erfinder: Marx, Thomas, 22525 Hamburg (DE); Schöller, Bernd, 76571 Gaggenau - Freiolsheim (DE)
(74) Vertreter: Marx, Thomas

(56) Entgegenhaltungen:
- EP-A- 1 702 649
- EP-A2- 0 601 589
- EP-A2- 1 757 226
- WO-A-01/87149
- WO-A-2005/050525
- WO-A2-2006/026335
- DE-A1-102005 013 852
- DE-A1-102005 049 643
- US-A1- 2002 077 856
- US-A1- 2005 245 830
- US-A1- 2006 093 785
- US-A1- 2010 071 696
- US-A1- 2010 292 544

## Beschreibung

Die Erfindung betrifft ein Verfahren zur Aktualisierung von Beatmungsgeräten, bei dem ein im Gerät abgespeichertes Betriebsprogramm mindestens teilweise durch ein neues Betriebsprogramm ersetzt wird.

Die Erfindung betrifft darüber hinaus eine Vorrichtung zur Aktualisierung von Beatmungsgeräten, die einen Speicher für ein Betriebsprogramm aufweist.

Bei den meisten Medizintechnik-Geräten wird die Betriebssoftware in einem Permanentspeicher abgelegt und von einem µController ausgeführt, der für diese Software ausgelegt ist. Folglich ist die Software in Form von nicht veränderbaren Programmanweisungen hinterlegt und der µController ist nicht erweiterbar bzw. hat keine freien ist nicht erweiterbar bzw. hat keine freien Kapazitäten. Im Falle eines Software-Updates müssen somit Speicherbauelemente ersetzt werden, was zur Folge hat, dass die Geräte von einem Fachmann demontiert werden müssen. Ein solches Software-Update ist aufwändig und teuer, da während der Dauer des Updateprozesses das betreffende Medizingerät nicht verwendet werden kann.

In anderen Fällen ist der Austausch von Hardware notwendig, um beispielsweise Sensoren mit erweiterter Funktionalität verwenden zu können.

Aufgrund der raschen Fortentwicklung der technischen Möglichkeiten sind Medizintechnik-Geräte häufig schon nach kurzer Nutzungsdauer nicht mehr auf dem Stand der Technik. Derzeit müssen Benutzer, die auf die neuesten oder weitere benötigte Funktionen angewiesen sind, daher regelmäßig ein neues Medizintechnik-Gerät erwerben.

Die DE 10 2005 049643 A1 beschreibt ein Beatmungsgerät mit einer Motorsteuerung, die einen Datenspeicher mit genügend freien Speicherplätzen oder einen ergänzten Datenspeicher zur Abspeicherung einer neuen Regelungssteuerung aufweist. In den freien oder ergänzten Datenspeicher sind solche Werte eingespeichert, die einen - gegenüber dem Standardbetrieb - veränderten Betrieb hinsichtlich Lärmabstrahlung oder Energieverbrauch des Gerätes oder hinsichtlich der Atemarbeit des Patienten bedingen.

Die EP 1 702 649 A offenbart ein medizintechnisches System zumindest bestehend aus einem Beatmungsgerät und einem Defibrillator, wobei diese miteinander koppelbar sind und dann Daten austauschen können.

Die WO 2005/050525 A beschreibt einen Patientenmonitor für den Einsatz im Krankenhaus, der physiologische Patientendaten von unterschiedlichen Sensoren empfangen und anzeigen kann und diese in einem Krankenhausnetzwerk verteilt.

Die WO 2006/026335 A2 beschreibt eine patientennahe Versorgungseinheit, die über eine Prozesseinheit angesteuert und versorgt wird, wobei ein bidirektionaler Datenaustausch vorgesehen ist.

Die US 2006/093785 A1 offenbart einen Anästhesie-Monitor mit einer Schnittstelle, über die Aktualisierungen der Software erfolgen können, oder Systeminformationen oder Patientendaten übertragen werden können.

Die DE 10 2005 013 852 A1 offenbart die Fernwartung eines medizintechnischen Gerätes, insbesondere eines Röntgengerätes, über das Stromnetz.

Die WO 2006/006159 A1 beschreibt ein übliches Verfahren zur Aktualisierung von Software.

Die US 2002/077856 A1 offenbart die Aktualisierung von Software und/oder Firmware bei einem Beatmungsgerät, um neue Beatmungssoftware aufzuspielen. Eine Aktualisierung des Beatmungsgerätes wird ermöglicht, wenn der interne Zugriffsschlüssel mit dem externen Zugriffsschlüssel übereinstimmt.

Es wäre wünschenswert, für Medizintechnik-Geräte eine update Funktionalität vorzusehen, die es dem Benutzer ermöglicht, sein Medizintechnik-Gerät mit den jeweils neuesten oder zusätzlichen benötigten Funktionen aufzurüsten.

Ein Medizintechnik-Gerät ist im Sinne der Erfindung ein Beatmungsgerät.

Aufgabe der vorliegenden Erfindung ist es, eine Vorrichtung und ein Verfahren anzugeben, die eine Aktualisierung der Software eines Beatmungsgerätes und eine schnelle Ausführung der neuen Software ermöglichen, die zudem sicherstellen, dass die Aktualisierung nicht den Algorithmus für die Ausführung der Betriebssoftware beschädigt und zudem ermöglichen, dass die Firmware des µControllers aktualisiert werden kann.

Diese Aufgabe wird erfindungsgemäß gelöstdurch ein Verfahren nach Anspruch 1 und eine Vorrichtung nach Anspruch 7.

Insbesondere wird die Aufgabe auch erfindungsgemäß dadurch gelöst, dass die erfindungsgemäße Vorrichtung für ein Medizintechnik-Gerät aus einer internen oder externen Eingangsvorrichtung für Dateneingabe und/oder Datenempfang besteht, sowie aus einer hiermit mindestens mittelbar verbundenen Speichervorrichtung, mit in der Speichervorrichtung in zumindest einem Speicher abgelegten Daten oder Rechen- bzw. Zählalgorithmen, sowie mit Prüfmitteln, die eingegebene oder empfangene Daten (hier: "Code") mit in einem oder mehreren Speichern der Speichervorrichtung abgelegten Daten automatisch vergleichen, insbesondere auch, nachdem oder während der Code und/oder die in einem oder mehreren Speichern abgelegten Daten verarbeitet wurden oder werden (insbesondere Rechen- bzw. Zählalgorithmen durchlaufen haben oder durchlaufen), wobei Steuerungsmittel vorhanden sind, die aufgrund des durch die Prüfmittel ermittelten Vergleichsergebnisses nur bei bestimmten definierten Code(s) selbsttätig veranlassen, dass das Gerät zumindest zeitweise bestimmte Funktionen ausführen kann, die diesen Codes zugeordnet sind, und dass die Ausführung dieser den Codes zugeordneten Funktionen ohne den entsprechenden Code durch diese Steuerungsmittel oder durch andere Mittel gesperrt ist.

Die Aufgabe wird erfindungsgemäß auch dadurch gelöst, dass das Bedienungsprogramm mindestens teilweise in einem veränderlichen Speicher des Gerätes gespeichert wird und bei der Durchführung der Aktualisierung mindestens teilweise durch das neue Betriebsprogramm ersetzt wird.

Gemäß einer Ausführungsform besteht die Vorrichtung für ein Medizintechnikgerät aus einer internen oder externen Eingangsvorrichtung für Daten- und/oder Rechenprogramm-Eingaben oder Daten- und/oder Rechenprogramm-Empfang, aus einer hiermit mindestens mittelbar verbundenen Speichervorrichtung, mit in der Speichervorrichtung in einem oder mehreren Speichern abgelegten Daten oder Rechen- bzw. Zählalgorithmen, sowie mit Prüfmitteln, die eingegebene oder empfangene Daten und/oder Rechenprogramme, hier als "Updates" bezeichnet, mit in einem oder mehreren Speichern der Speichervorrichtung abgelegten Daten automatisch vergleichen, insbesondere auch, nachdem oder während das Update und/oder die in einem oder mehreren Speichern abgelegten Daten verarbeitet wurden oder werden und insbesondere Rechen- bzw. Zählalgorithmen durchlaufen haben oder durchlaufen, wobei Steuerungsmittel vorhanden sind, die aufgrund des durch die Prüfmittel ermittelten Vergleichsergebnisses nur bei bestimmten definierten Updates selbsttätig veranlassen, dass das Gerät dauerhaft oder zeitweise diese Daten, Programme oder hieraus berechnete Daten oder Programme auf Hardwarefunktionen anwendet, die diesen Updates zugeordnet sind, und dass die Ausführung dieser den Updates zugeordneten Hardwarefunktionen ohne das entsprechende Update durch diese Steuerungsmittel oder durch andere Mittel gesperrt ist.

Betriebsprogramme im Sinne der Erfindung sind beispielsweise die Betriebssoftware für die Gerätefunktion, Anwendungsprogramme, Auswertungsprogramme für Messwerte oder ein Kommunikationsprogramm.

Gemäß einer weiteren Ausführungsform besteht die Vorrichtung für ein Medizintechnik-Gerät aus einer internen oder externen Eingangsvorrichtung für Dateneingaben oder Datenempfang, aus einer hiermit mindestens mittelbar verbundenen Speichervorrichtung, mit in der Speichervorrichtung in einem oder mehreren Speichern abgelegten Daten oder Rechen- bzw. Zählalgorithmen, sowie mit Prüfmitteln, die eingegebene oder empfangene Daten, hier: "Code", mit in einem oder mehreren Speichern der Speichervorrichtung abgelegten Daten automatisch vergleichen, insbesondere auch, nachdem oder während der Code und/oder die in einem oder mehreren Speichern abgelegten Daten verarbeitet wurden oder werden, wobei Steuerungsmittel vorhanden sind, die aufgrund des durch die Prüfmittel ermittelten Vergleichsergebnisses nur bei bestimmten definierten Code(s) selbsttätig veranlassen, dass das Gerät dauerhaft oder zeitweise mit einer oder mehreren bestimmten zusätzlichen internen oder externen Hardwarekomponenten zusammenarbeitet, die diesen Codes zugeordnet sind, und dass die Zusammenarbeit des Gerätes mit einer den Codes zugeordneten Hardwarekomponente ohne den entsprechenden Code durch diese Steuerungsmittel oder durch andere Mittel gesperrt ist.

Eine für den Benutzer optimierte Funktionalität wird dadurch erreicht, dass ein positives Vergleichsergebnis akustisch, optisch oder auf andere Weise dem Benutzer angezeigt oder gemeldet wird, z.B. als Erfolgsmeldung.

Eine für den Benutzer optimierte Funktionalität wird auch dadurch erreicht, dass ein negatives Vergleichsergebnis akustisch, optisch oder auf andere Weise dem Benutzer angezeigt oder gemeldet wird, z.B. als Fehlermeldung.

Eine erweiterte Funktionalität wird dadurch erreicht, dass in der Speichervorrichtung und/oder in dem Code Daten abgelegt sind, die, auch in verarbeiteter oder berechneter Form, die Ausführung der Funktionen und/oder deren Sperrung zeitlich in einer definierten Weise begrenzen.

Eine individuell anpassbare Verwendung wird dadurch erreicht, dass Hardwarekomponenten im Gerät enthalten sind, die ohne Code-Eingabe nicht in Funktion genommen werden können und insoweit für alle Funktionen gesperrt sind.

Eine konstruktiv einfache Realisierung wird dadurch erreicht, dass ohne die Code-Eingabe die Versorgung des Gerätes oder von Teilen hiervon mit elektrischer Energie (z.B. über Netzverbindung, Batterien oder Akkus) unterbunden, oder zeitmäßig oder leitungsmäßig in definierter Weise begrenzt wird.

Eine schnelle Realisierung der Freigabe wird dadurch erreicht, dass die Eingabe oder Übermittlung des Codes an die Eingangsvorrichtung mittels eines Datenträgers mit patienten- oder praxis- oder arztbezogenen Daten, wie über z.B. die Gesundheitskarte, oder über ein Lesegerät hierfür erfolgt.

Eine wirkungsvolle Sperrung wird dadurch erreicht, dass ohne die Code-Eingabe bestimmte Bauteile des Gerätes, insbesondere Sensoren bestimmter Kenndaten, zumindest zeitweise zur Nutzung für das Gerät gesperrt sind.

Eine erweiterte Funktionalität wird dadurch bereitgestellt, dass in der Speichervorrichtung und/oder in dem Code Daten abgelegt sind, die, auch in verarbeiteter oder berechneter Form, die Ausführung der den Updates zugeordneten Hardwarefunktionen und/oder deren Sperrung zeitlich in einer definierten Weise begrenzen.

Eine konstruktiv einfache Realisierung wird dadurch erreicht, dass Hardwarekomponenten im Gerät enthalten sind, die ohne das Update nicht in Funktion genommen werden können und insoweit für alle Funktionen gesperrt sind.

Erfindungsgemäß sind ohne das Update bestimmte Bauteile des Gerätes, insbesondere Sensoren bestimmter Kenndaten, wie beispielsweise Wellenlängen, zumindest zeitweise zur Nutzung für das Gerät gesperrt.

Erfindungsgemäß sind in der Speichervorrichtung und/oder in dem Code Daten abgelegt, die die Zusammenarbeit des Gerätes mit der zugeordneten zusätzlichen Hardwarekomponente und/oder deren Sperrung zeitlich in einer definierten Weise begrenzen.

Erfindungsgemäß sind alternativ und / oder ergänzend bereits Hard- oder Softwarekomponenten im Gerät enthalten, die ohne die Zusammenarbeit des Gerätes mit der zugeordneten zusätzlichen Hardwarekomponente nicht in Funktion genommen werden können und insoweit für alle Funktionen gesperrt sind.

Eine gerätetechnische Ausführungsform der Erfindung ist dadurch realisiert, dass die ohne Code gesperrten, bereits vorhandenen Hardwarekomponenten und/oder die von zusätzlichen Hardwarekomponenten definierte Sensoren, insbesondere von bestimmter Wellenlänge sind.

Eine konstruktiv einfache Variante sieht vor, dass der Code in der zusätzlichen Hardwarekomponente und/oder in den Mitteln gespeichert ist, die die zusätzliche Hardwarekomponente mit dem Gerät verbinden und durch die Herstellung der Verbindung zwischen dieser Hardwarekomponente und dem Gerätes an die Eingangsvorrichtung gelangt.

Eine erhöhte Funktionalität wird dadurch bereitgestellt, dass eine optische oder akustische oder mechanische Anzeige/Signalausgabe vorhanden ist, die für die Freischaltung durch Code, das Upgrade oder die zusätzliche Hardwarekomponente Anzeige/Ausgabemöglichkeiten bereit hält, die ohne die Freischaltung durch Code, das Upgrade oder die zusätzliche Hardwarekomponente nicht genutzt werden können.

Ein Schutz vor unsachgemäßer Nutzung wird dadurch realisiert, dass eine definierte mehrmalige Eingabe eines oder mehrerer falscher Codes oder eines oder mehrerer nicht passender Updates zu einer zeitlich begrenzten oder unbegrenzten Sperre der Annahme jeder weiteren Eingabe für Codes oder Updates führt, welche ggf. nur mit einem weiteren Code entsperrt werden kann (der z. B. nur dem Hersteller bekannt ist).

Gemäß einer Ausführungsform kann die Erfindung als ein Verfahren zur Aktualisierung von Software und/oder Firmware bei elektronischen Medizingeräten realisiert sein, wobei die Medizingeräte eine wiederbeschreibbare Speichereinheit aufweisen, zum Speichern einer aktualisierten und/oder einer vorhergehenden Version der Software und/oder Firmware, die Schritte enthaltend:
(a) Bereitstellen der Software und/oder Firmware zur Aktualisierung mit Hilfe eines Aktualisierungsmittels;
(b) Kommunikation des Medizingerätes mit dem Aktualisierungsmittel;
(c) bei erfolgter Kommunikation: Übertragen der Aktualisierung vom Aktualisierungsmittel auf das Medizingerät;
(d) Schreiben der Aktualisierung in eine wiederbeschreibbare Speichereinheit;
(e) Verifizierung der Aktualisierung als gültige Version und Identifizierung der vorhergehenden Version als ungültig;
(f) Ausführen der aktualisierten Software und/oder Firmware.

Erfindungsgemäß ist daran gedacht, dass die vorhergehende Version mit der aktualisierten Software und/oder Firmware überschrieben oder in einen Restore-Speicher verschoben wird, insbesondere, wenn die aktualisierte Form nur für eine begrenzte Zeit freigeschaltet ist.

Erfindungsgemäß ist auch daran gedacht, dass das Medizintechnikgerät zusätzlich einen Permanentspeicher einschließlich Anweisungen enthält, welche nach der Aktualisierung ausgeführt werden und woraufhin die wiederbeschreibbare Speichereinheit nach einer aktualisierten Software und/oder Firmware durchsucht wird und diese anschließend geladen und ausgeführt wird.

Gemäß einer weiteren Ausführungsform kann die Erfindung als ein Verfahren zur Aktualisierung von Software und/oder Firmware bei elektronischen Medizingeräten realisiert sein, wobei die Medizingeräte einen µController mit einer Speichereinheit aufweisen und der µController über eine Datenverbindung, beispielsweise über freie PINs, wie folgt angesteuert werden kann:
(a) Senden eines Signals an den µController, um einen Reset des µControllers zu bewirken, beispielsweise über freie PINs;
(b) Bereitstellen eines Flash-Programms mit neuer Firmware zum Schreiben der neuen Firmware auf den µController,
(c) wobei der Programmeintrittspunkt an eine definierte Stelle des µControllers geschrieben wird, welche nach Ende des Resets zuerst ausgelesen wird.

In den Zeichnungen sind Ausführungsbeispiele der Erfindung schematisch dargestellt. Es zeigt:

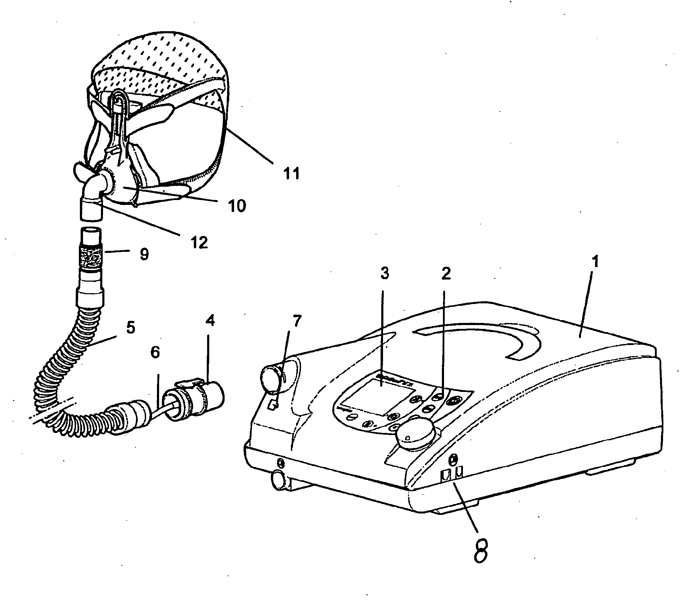

Fig. 1 eine perspektivische Darstellung eines Beatmungsgerätes mit Verbindungsschlauch zu einer Beatmungsmaske.

Fig. 1 zeigt den grundsätzlichen Aufbau eines Beatmungsgerätes. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Über die Schnittstelle können weitere Medizintechnische Geräte angeschlossen werden. Beispielsweise können Pulsoximeter oder Pulsspektrometer angeschlossen werden. Ein Anfeuchter kann ebenfalls adaptiert werden.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus ein als Beatmungsmaske (10) ausgebildetes Patienten-Interface, das als Nasalmaske realisiert ist. Eine Fixierung im Bereich des Kopfes eines Patienten kann über eine Kopfhaube (11) erfolgen. Im Bereich ihrer dem Verbindungsschlauch (5) zugewandten Ausdehnung weist das Patienten-Interface (10) ein Kupplungselement (12) auf.

Über die Schnittstelle (8) kann die Eingabe und / oder Ausgabe von Daten, wie beispielsweise eine Eingabe des Totraumvolumens oder ein Softwareupdate / Firmware-Update, erfolgen. Die Schnittstellen können beispielsweise kabelgebunden, als Infrarot-Schnittstelle, als Bluetooth-Schnittstelle oder USB realisiert sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Zudem können im Gerät Schnittstellen für adaptierbare Zusatzgeräte und Informations-Management-Systeme, beispielsweise zur Aufnahme von Speichermedien, oder zur Verbindung mit einem EKG, EEG, Drucker, Defibrillator, Pulsoximeter oder einem anderen medizintechnischen Gerät vorgesehen werden.

Über ein Modem oder eine andere Schnittstelle können ebenfalls aufgezeichnete Daten, wie Trends, außerordentliche Ereignisse, Warnmeldungen etc. dem Arzt, sowie Auffälligkeiten, Betriebsstunden oder andere zur Gewährleistung der einwandfreien Funktion dem Nutzer oder dem Wartungs-/Kundendienst bei Bedarf übermittelt werden.

Es ist ebenfalls möglich, über eine Schnittstelle Sensoren für die Ermittlung weiterer Körperparameter zu adaptieren. So können beispielsweise adaptierbare Zusatzgeräte wie EKG, EEG, EMG, EOG, Pulsoximeter, Pulsspektrometer ergänzt werden. Die von den adaptierten Sensoren ermittelten Körperparameter können auf dem Display des Beatmungsgerätes angezeigt werden. Dafür sind im Bereich des Displays solche Bereiche vorgesehen, die erst dann aktiviert werden, wenn der betreffende Sensor adaptiert ist. In diesen Bereichen werden dann Messwerte der adaptierten Sensoren angezeigt.

Über die Eingangsvorrichtung für Dateneingabe / Datenempfang, beispielsweise als Tastenfeld ausgeführt, kann ein Code eingegeben werden, der der Freigabe von Gerätefunktionen dient.

Nach der Code-Eingabe wird ein positives Vergleichsergebnis akustisch oder optisch, beispielsweise durch eine grün leuchtende LED, dem Benutzer als Erfolgsmeldung gemeldet.

Ein negatives Vergleichsergebnis wird akustisch oder optisch, beispielsweise durch eine rot leuchtende LED, dem Benutzer als Fehlermeldung gemeldet.

Im Sinne der Erfindung ist auch daran gedacht, ein Beatmungsgerät über eine Schnittstelle zumindest zeitweise mit einem adaptierbaren Zusatzgerät zu verbinden, beispielsweise mit einem EKG, EEG, EOG, EMG, Drucker, Monitor, Defibrillator, Pulsoximeter oder einem anderen medizintechnischen Gerät. Hierbei ist ebenfalls eine Freigabe des adaptierbaren Zusatzgerätes durch eine Code-Eingabe vorgesehen.

## Patentansprüche

1. Verfahren zur Aktualisierung von Software und/oder Firmware bei elektronischen Medizingeräten, wobei das Medizingerät ein Beatmungsgerät ist bei dem im Bereich eines Gerätegehäuses (1) mit Bedienfeld (2) sowie Anzeige (3) in einem Geräteinnenraum eine Atemgaspumpe angeordnet ist und über eine Kopplung (4) ein Verbindungsschlauch (5) angeschlossen ist und zur Ermöglichung einer Datenübertragung das Gerätegehäuse (1) eine Schnittstelle (8) aufweist über die die Eingabe und / oder Ausgabe von Daten erfolgt und das Beatmungsgerät eine wiederbeschreibbare Speichereinheit aufweist, zum Speichern einer aktualisierten Version der Software und/oder Firmware, die Schritte enthaltend:
(a) Bereitstellen der Software und/oder Firmware zur Aktualisierung mit Hilfe eines Aktualisierungsmittels;
(b) Kommunikation des Beatmungsgerätes mit dem Aktualisierungsmittel;
(c) bei erfolgter Kommunikation: Übertragen der Aktualisierung vom Aktualisierungsmittel auf das Beatmungsgerät;
(d) Schreiben der Aktualisierung in eine wiederbeschreibbare Speichereinheit;
(e) Verifizierung der Aktualisierung als gültige Version und Identifizierung der vorhergehenden Version als ungültig;
(f) Ausführen der aktualisierten Software und/oder Firmware **dadurch gekennzeichnet, dass** über ein Modem oder eine andere Schnittstelle aufgezeichnete Daten dem Arzt, sowie Auffälligkeiten, Betriebsstunden oder andere zur Gewährleistung der einwandfreien Funktion dem Nutzer oder dem Wartungs-/Kundendienst bei Bedarf übermittelt werden, wobei das Beatmungsgerät zusätzlich einen Permanentspeicher einschließlich Anweisungen enthält, welche nach der Aktualisierung ausgeführt werden und woraufhin die wiederbeschreibbare Speichereinheit nach einer aktualisierten Software und/oder Firmware durchsucht wird und diese anschließend geladen und ausgeführt wird, wobei das Beatmungsgerät einen µController mit einer Speichereinheit aufweist und der µController über eine Datenverbindung wie folgt angesteuert werden kann: (a) Senden eines Signals an den µController, um einen Reset des µControllers zu bewirken; (b) Bereitstellen eines Flash-Programms mit neuer Firmware zum Schreiben der neuen Firmware auf den µController, (c) wobei der Programmeintrittspunkt an eine definierte Stelle des µControllers geschrieben wird, welche nach Ende des Resets zuerst ausgelesen wird.

2. Verfahren nach Anspruch 1. **dadurch gekennzeichnet, dass** über die Schnittstelle weitere medizintechnische Geräte anschließbar sind.

3. Verfahren nach Anspruch 1. oder 2. **dadurch gekennzeichnet, dass** das Beatmungsgerät über eine Schnittstelle (8) zumindest zeitweise mit einem Defibrillator, verbunden ist, wobei eine Freigabe des Defibrillators durch eine Code-Eingabe erfolgt.

4. Verfahren nach wenigstens einem der vorhergehenden Ansprüche **dadurch gekennzeichnet, dass** im Beatmungsgerät Schnittstellen für adaptierbare Zusatzgeräte und Informations-Management-Systeme, zur Aufnahme von Speichermedien, oder zur Verbindung mit einem EKG, EEG, Drucker, Defibrillator, Pulsoximeter oder einem anderen medizintechnischen Gerät vorgesehen sind.

5. Verfahren nach wenigstens einem der vorhergehenden Ansprüche **dadurch gekennzeichnet, dass** über eine Schnittstelle Sensoren für die Ermittlung weiterer Körperparameter adaptiert sind wobei die von den adaptierten Sensoren ermittelten Körperparameter auf dem Display des Beatmungsgerätes angezeigt werden wofür im Bereich des Displays solche Bereiche vorgesehen sind, die erst dann aktiviert werden, wenn der betreffende Sensor adaptiert ist und in diesen Bereichen dann Messwerte der adaptierten Sensoren angezeigt werden.

6. Verfahren nach wenigstens einem der vorhergehenden Ansprüche **dadurch gekennzeichnet, dass** der Speicher mindestens bereichsweise als veränderlicher Speicher ausgebildet und mit einer Eingabeeinrichtung für ein aktuelles Betriebsprogramm verbunden ist und dass der Speicher und die Eingabeeinrichtung an Steuerungsmittel angeschlossen sind, die Prüfmittel zur Auswertung mindestens eines Code aufweisen, wobei mindestens eine Funktion des Gerätes nur bei einer Übereinstimmung des Codes mit einem Referenzwert freigeschaltet ist.

7. Vorrichtung für ein Medizintechnikgerät bestehend aus einer internen oder externen Eingangsvorrichtung für DatenEingaben oder Daten-Empfang, aus einer hiermit mindestens mittelbar verbundenen Speichervorrichtung, mit in der Speichervorrichtung in einem Speicher abgelegten Daten, sowie mit Prüfmitteln, die eingegebene oder empfangene Daten, hier als "Updates" bezeichnet, mit in einem Speicher der Speichervorrichtung abgelegten Daten automatisch vergleichen, insbesondere auch, nachdem oder während das Update und/oder die in einem oder mehreren Speichern abgelegten Daten verarbeitet wurden und insbesondere Rechen- bzw. Zählalgorithmen durchlaufen haben, wobei Steuerungsmittel vorhanden sind, die aufgrund des durch die Prüfmittel ermittelten Vergleichsergebnisses nur bei bestimmten definierten Updates selbsttätig veranlassen, dass das Beatmungsgerät dauerhaft oder zeitweise diese Daten, Programme auf Hardwarefunktionen anwendet die diesen Updates zugeordnet sind und, dass die Ausführung dieser den Updates zugeordneten Hardwarefunktionen ohne das entsprechende Update durch diese Steuerungsmittel oder durch andere Mittel gesperrt ist, wobei das Medizintechnik-Gerät ein Beatmungsgerät ist bei dem im Bereich eines Gerätegehäuses (1) mit Bedienfeld (2) sowie Anzeige (3) in einem Geräteinnenraum eine Atemgaspumpe angeordnet ist und über eine Kopplung (4) ein Verbindungsschlauch (5) angeschlossen ist und zur Ermöglichung einer Datenübertragung das Gerätegehäuse (1) eine Schnittstelle (8) aufweist über die die Eingabe und / oder Ausgabe von Daten erfolgt **dadurch gekennzeichnet, dass** über ein Modem oder eine andere Schnittstelle aufgezeichnete Daten dem Arzt, sowie Auffälligkeiten, Betriebsstunden oder andere zur Gewährleistung der einwandfreien Funktion dem Nutzer oder dem Wartungs-/Kundendienst bei Bedarf übermittelt werden, wobei das Beatmungsgerät zusätzlich einen Permanentspeicher einschließlich Anweisungen enthält, welche nach der Aktualisierung ausgeführt werden und woraufhin die wiederbeschreibbare Speichereinheit nach einer aktualisierten Software und/oder Firmware durchsucht wird und diese anschließend geladen und ausgeführt wird, wobei das Beatmungsgerät einen µController mit einer Speichereinheit aufweist und der µController über eine Datenverbindung wie folgt angesteuert werden kann: (a) Senden eines Signals an den µController, um einen Reset des µControllers zu bewirken; (b) Bereitstellen eines Flash-Programms mit neuer Firmware zum Schreiben der neuen Firmware auf den µController, (c) wobei der Programmeintrittspunkt an eine definierte Stelle des µControllers geschrieben wird, welche nach Ende des Resets zuerst ausgelesen wird.

8. Vorrichtung nach Anspruch 7. **dadurch gekennzeichnet, dass** über die Schnittstelle weitere medizintechnische Geräte anschließbar sind.

9. Vorrichtung nach Anspruch 7. oder 8. **dadurch gekennzeichnet, dass** das Beatmungsgerät über eine Schnittstelle zumindest zeitweise mit einem Defibrillator verbunden ist, wobei eine Freigabe des Defibrillators durch eine Code-Eingabe erfolgt.

10. Vorrichtung nach wenigstens einem der vorhergehenden Ansprüche **dadurch gekennzeichnet, dass** im Beatmungsgerät Schnittstellen für adaptierbare Zusatzgeräte und Informations-Management-Systeme, zur Aufnahme von Speichermedien, oder zur Verbindung mit einem EKG, EEG, Drucker, Defibrillator, Pulsoximeter oder einem anderen medizintechnischen Gerät vorgesehen sind.

11. Vorrichtung nach wenigstens einem der vorhergehenden Ansprüche **dadurch gekennzeichnet, dass** über eine Schnittstelle Sensoren für die Ermittlung weiterer Körperparameter adaptierbar sind wobei die von den adaptierten Sensoren ermittelten Körperparameter auf dem Display des Beatmungsgerätes angezeigt werden wofür im Bereich des Displays solche Bereiche vorgesehen sind, die erst dann aktiviert werden, wenn der betreffende Sensor adaptiert ist und in diesen Bereichen dann Messwerte der adaptierten Sensoren angezeigt werden.

12. Vorrichtung nach wenigstens einem der vorhergehenden Ansprüche **dadurch gekennzeichnet, dass** der Speicher mindestens bereichsweise als veränderlicher Speicher ausgebildet und mit einer Eingabeeinrichtung für ein aktuelles Betriebsprogramm verbunden ist und dass der Speicher und die Eingabeeinrichtung an Steuerungsmittel angeschlossen sind, die Prüfmittel zur Auswertung mindestens eines Codes aufweisen, wobei mindestens eine Funktion des Gerätes nur bei einer Übereinstimmung des Codes mit einem Referenzwert freigeschaltet ist.

## Claims

1. A method for updating software and/or firmware in electronic medical appliances, wherein the medical appliance is a ventilator in which, in the area of an appliance housing (1) comprising a control panel (2) and a display (3), a breathing gas pump is arranged in an appliance interior and a connection tube (5) is connected via a coupling (4) and, for enabling data transmission, the appliance housing (1) has an interface (8) via which data are input and/or output and the ventilator has a rewritable storage unit for storing an updated version of the software and/or firmware, containing the steps of:
(a) providing the software and/or firmware for updating with the aid of an update means;
(b) the ventilator communicating with the update means;
(c) if communication has taken place: transmitting the update from the update means to the ventilator;
(d) writing the update to a rewritable storage unit;
(e) verifying the update as a valid version and identifying the previous version as invalid;
(f) running the updated software and/or firmware,
**characterized in that**, via a modem or another interface, recorded data are transmitted to the doctor, and abnormalities, operating hours or other data for ensuring proper function are transmitted to the user or to the maintenance/customer service, as required, wherein the ventilator additionally contains a permanent storage, including instructions which are carried out after the update and whereupon the rewritable storage unit is searched for updated software and/or firmware which is subsequently loaded and run, wherein the ventilator has a microcontroller comprising a storage unit and the microcontroller can be controlled via a data connection, as follows: (a) sending a signal to the microcontroller to cause the microcontroller to reset; (b) providing a flash program containing new firmware for writing the new firmware to the microcontroller, (c) wherein the program entry point is written at a defined position in the microcontroller, which position is first read out when the reset has ended.

2. The method according to claim 1, **characterized in that** further medical appliances can be connected via the interface.

3. The method according to claim 1 or claim 2, **characterized in that** the ventilator is connected, via an interface (8), at least temporarily to a defibrillator, wherein the defibrillator is enabled by inputting a code.

4. The method according to at least one of the preceding claims, **characterized in that** interfaces for adaptable auxiliary appliances and information management systems, for receiving storage media or for connection to an ECG, EEG, printer, defibrillator, pulse oximeter or another medical appliance, are provided in the ventilator.

5. The method according to at least one of the preceding claims, **characterized in that** sensors for determining further body parameters are adapted via an interface, wherein the body parameters determined by the adapted sensors are shown on the display on the ventilator, for which purpose such areas are provided in the area of the display that are only activated when the relevant sensor is adapted, and measured values from the adapted sensors are then shown in these areas.

6. The method according to at least one of the preceding claims, **characterized in that** the storage is designed at least in certain areas as a variable storage and is connected to an input apparatus for a current operating program, and **in that** the storage and the input apparatus are connected to control means that have testing means for evaluating at least one code, wherein at least one function of the appliance is enabled only when the code corresponds to a reference value.

7. A device for a medical appliance, consisting of an internal or external input device for data inputs or data reception, and of a storage device connected at least indirectly thereto, containing data saved in a storage in the storage device, and comprising testing means that automatically compare input or received data, referred to here as "updates", with data saved in a storage of the storage device, in particular also after or while the update and/or the data saved in one or more storages were processed, and in particular underwent calculation algorithms or counting algorithms, wherein control means are present which, on the basis of the comparison result determined by the testing means only in the case of specific, defined updates, independently cause the ventilator to permanently or temporarily apply these data or programs to hardware functions associated with these updates, and cause these hardware functions associated with the updates to be executed without the corresponding update being blocked by these control means or by other means, wherein the medical appliance is a ventilator in which, in the area of an appliance housing (1) comprising a control panel (2) and a display (3), a breathing gas pump is arranged in an appliance interior and a connection tube (5) is connected via a coupling (4) and, for enabling data transmission, the appliance housing (1) has an interface (8) via which data are input and/or output, **characterized in that**, via a modem or another interface, recorded data are transmitted to the doctor, and abnormalities, operating hours or other data for ensuring proper function are transmitted to the user or to the maintenance/customer service, as required, wherein the ventilator additionally contains a permanent storage, including instructions which are carried out after the update and whereupon the rewritable storage unit is searched for updated software and/or firmware which is subsequently loaded and run, wherein the ventilator has a microcontroller comprising a storage unit and the microcontroller can be controlled via a data connection, as follows: (a) sending a signal to the microcontroller to cause the microcontroller to reset; (b) providing a flash program containing new firmware for writing the new firmware to the microcontroller, (c) wherein the program entry point is written at a defined position in the microcontroller, which position is first read out when the reset has ended.

8. The device according to claim 7, **characterized in that** further medical appliances can be connected via the interface.

9. The device according to claim 7 or claim 8, **characterized in that** the ventilator is connected, via an interface, at least temporarily to a defibrillator, wherein the defibrillator is enabled by inputting a code.

10. The device according to at least one of the preceding claims, **characterized in that** interfaces for adaptable auxiliary appliances and information management systems, for receiving storage media or for connection to an ECG, EEG, printer, defibrillator, pulse oximeter or another medical appliance, are provided in the ventilator.

11. The device according to at least one of the preceding claims, **characterized in that** sensors for determining further body parameters can be adapted via an interface, wherein the body parameters determined by the adapted sensors are shown on the display of the ventilator, for which purpose such areas are provided in the area of the display that are only activated when the relevant sensor is adapted, and measured values from the adapted sensors are then shown in these areas.

12. The device according to at least one of the preceding claims, **characterized in that** the storage is designed at least in certain areas as a variable storage and is connected to an input apparatus for a current operating program, and **in that** the storage and the input apparatus are connected to control means that have testing means for evaluating at least one code, wherein at least one function of the appliance is enabled only when the code corresponds to a reference value.

## Revendications

1. Procédé d'actualisation du logiciel et/ou du microprogramme d'appareils médicaux électroniques, dans lequel l'appareil médical est un respirateur, sur lequel une pompe d'alimentation en gaz respiratoire est disposée au niveau d'un boîtier d'appareil (1) doté d'un panneau de commande (2) ainsi que d'un afficheur (3) dans un espace intérieur de l'appareil et un tuyau de raccordement (5) est branché au moyen d'un élément de couplage (4) et dans lequel le boîtier d'appareil (1) présente une interface (8) permettant une transmission de données, par laquelle l'entrée et / ou la sortie des données s'effectuent et le respirateur comporte une unité de mémoire réinscriptible pour enregistrer une version actualisée du logiciel et/ou du microprogramme, comprenant les étapes suivantes :
(a) fourniture du logiciel et/ou du microprogramme pour mettre à jour à l'aide d'un moyen d'actualisation ;
(b) communication du respirateur avec le moyen d'actualisation ;
(c) une fois la communication réalisée : transmission de la mise à jour du moyen d'actualisation vers le respirateur ;
(d) transcription de la mise à jour dans une unité de mémoire réinscriptible ;
(e) vérification de la mise à jour comme version valide et identification de la version précédente comme invalide ;
(f) exécution du logiciel et/ou du microprogramme actualisés
**caractérisé en ce que** des données enregistrées sont transmises au médecin de même que des anomalies, des heures de service ou d'autres renseignements servant à garantir le bon fonctionnement sont communiqués à l'utilisateur ou au service de maintenance / à la clientèle via un modem ou une autre interface en cas de besoin, dans lequel le respirateur comporte en plus une mémoire permanente y compris des instructions, qui sont exécutées après la mise à jour et à la suite de quoi l'unité de mémoire réinscriptible est explorée à la recherche d'un logiciel et/ou d'un microprogramme actualisés et ce dernier est ensuite chargé et exécuté, dans lequel le respirateur présente un microcontrôleur avec une unité de mémoire et le microcontrôleur peut être activé via une liaison de transmission de données comme suit : (a) émission d'un signal destiné au microcontrôleur pour induire une réinitialisation du microcontrôleur ; (b) fourniture d'un programme flash doté d'un nouveau microprogramme pour transcrire le nouveau microprogramme sur le microcontrôleur, (c) dans lequel le point d'entrée du programme est transcrit à un endroit défini du microcontrôleur, lequel est lu d'abord à l'issue de la réinitialisation.

2. Procédé selon la revendication 1 **caractérisé en ce que** d'autres appareils médicaux peuvent être raccordés via l'interface.

3. Procédé selon la revendication 1 ou 2 **caractérisé en ce que** le respirateur est relié au moins temporairement à un défibrillateur via une interface (8), dans lequel un déblocage du défibrillateur est réalisé par la saisie d'un code.

4. Procédé selon au moins l'une des revendications précédentes **caractérisé en ce que** des interfaces sont prévues dans le respirateur pour des accessoires adaptables et des systèmes de gestion d'informations, pour recevoir des supports de stockage ou pour être reliées à un ECG (*électrocardiogramme*), à un EEG (*électroencéphalogramme*), à une imprimante, à un défibrillateur, à un oxymètre de pouls ou à un autre appareil médical.

5. Procédé selon au moins l'une des revendications précédentes **caractérisé en ce que** des capteurs de détection d'autres paramètres corporels sont adaptés via une interface, dans lequel les paramètres corporels détectés par les capteurs adaptés sont visualisés sur l'afficheur du respirateur, moyennant quoi des zones sont prévues au niveau de l'afficheur, qui ne sont activées qu'une fois que le capteur concerné est adapté, et les valeurs de mesure des capteurs adaptés sont ensuite visualisées dans ces zones.

6. Procédé selon au moins l'une des revendications précédentes **caractérisé en ce que** la mémoire est conçue au moins partiellement sous la forme d'une mémoire modifiable et est reliée à une unité d'entrée pour un programme d'exploitation actuel et que la mémoire et l'unité d'entrée sont raccordées à des moyens de commande, qui présentent des moyens de vérification pour analyser au moins un code, dans lequel au moins une fonction de l'appareil ne peut être activée qu'en cas de concordance du code avec une valeur de référence.

7. Dispositif destiné à un appareil médical comportant un dispositif d'entrée interne ou externe destiné à saisir ou à recevoir des données, un dispositif de mémorisation y étant relié au moins indirectement, avec des données stockées dans une mémoire à l'intérieur du dispositif de mémorisation, ainsi qu'avec des moyens de vérification, qui comparent automatiquement des données saisies ou reçues, appelées en l'occurrence « Mises à jour », avec les données stockées dans une mémoire du dispositif de mémorisation, notamment aussi après ou pendant que la mise à jour et/ou les données stockées dans une ou plusieurs mémoires ont été traitées et en particulier soumises à des algorithmes de calcul et de comptage, dans lequel des moyens de commande sont disponibles, qui font en sorte automatiquement sur la base des résultats de la comparaison déterminés par les moyens de vérification uniquement en présence de certaines mises à jours définies que le respirateur applique durablement ou temporairement ces données et programmes à des fonctions matérielles, qui sont assignées à ces mises à jour et que l'exécution de ces fonctions matérielles associées aux mises à jour est bloquée sans la mise à jour correspondante par ces moyens de commande ou par d'autres moyens, dans lequel l'appareil médical est un respirateur, sur lequel une pompe d'alimentation en gaz respiratoire est disposée au niveau d'un boîtier d'appareil (1) doté d'un panneau de commande (2) ainsi que d'un afficheur (3) dans un espace intérieur de l'appareil et un tuyau de raccordement (5) est branché au moyen d'un élément de couplage (4) et dans lequel le boîtier d'appareil (1) présente une interface (8) permettant une transmission de données, par laquelle l'entrée et / ou la sortie des données s'effectuent, **caractérisé en ce que** des données enregistrées sont transmises au médecin de même que des anomalies, des heures de service ou d'autres renseignements servant à garantir le bon fonctionnement sont communiqués à l'utilisateur ou au service de maintenance / à la clientèle via un modem ou une autre interface en cas de besoin, dans lequel le respirateur comporte en plus une mémoire permanente y compris des instructions, qui sont exécutées après la mise à jour et à la suite de quoi l'unité de mémoire réinscriptible est explorée à la recherche d'un logiciel et/ou d'un microprogramme actualisés et ce dernier est ensuite chargé et exécuté, dans lequel le respirateur présente un microcontrôleur avec une unité de mémoire et le microcontrôleur peut être activé via une liaison de transmission de données comme suit : (a) émission d'un signal destiné au microcontrôleur pour induire une réinitialisation du microcontrôleur ; (b) fourniture d'un programme flash doté d'un nouveau microprogramme pour transcrire le nouveau microprogramme sur le microcontrôleur, (c) dans lequel le point d'entrée du programme est transcrit à un endroit défini du microcontrôleur, lequel est lu d'abord à l'issue de la réinitialisation.

8. Dispositif selon la revendication 7 **caractérisé en ce que** d'autres appareils médicaux peuvent être raccordés via l'interface.

9. Dispositif selon la revendication 7 ou 8 **caractérisé en ce que** le respirateur est relié au moins temporairement à un défibrillateur via une interface (8), dans lequel un déblocage du défibrillateur est réalisé par la saisie d'un code.

10. Dispositif selon au moins l'une des revendications précédentes **caractérisé en ce que** des interfaces sont prévues dans le respirateur pour des accessoires adaptables et des systèmes de gestion d'informations, pour recevoir des supports de stockage ou pour être reliées à un ECG, à un EEG, à une imprimante, à un défibrillateur, à un oxymètre de pouls ou à un autre appareil médical.

11. Dispositif selon au moins l'une des revendications précédentes **caractérisé en ce que** des capteurs de détection d'autres paramètres corporels sont adaptables via une interface, dans lequel les paramètres corporels détectés par les capteurs adaptés sont visualisés sur l'afficheur du respirateur, moyennant quoi des zones sont prévues au niveau de l'afficheur, qui ne sont activées qu'une fois que le capteur concerné est adapté, et les valeurs de mesure des capteurs adaptés sont ensuite visualisées dans ces zones.

12. Dispositif selon au moins l'une des revendications précédentes **caractérisé en ce que** la mémoire est conçue au moins partiellement sous la forme d'une mémoire modifiable et est reliée à une unité d'entrée pour un programme d'exploitation actuel et que la mémoire et l'unité d'entrée sont raccordées à des moyens de commande, qui présentent des moyens de vérification pour analyser au moins un code, dans lequel au moins une fonction de l'appareil ne peut être activée qu'en cas de concordance du code avec une valeur de référence.
